(19) Europäisches Patentamt
European Patent Office
Office européen des brevets

(11) EP 1 559 415 A1

(12) **EUROPEAN PATENT APPLICATION**
published in accordance with Art. 158(3) EPC

(43) Date of publication:
**03.08.2005 Bulletin 2005/31**

(21) Application number: **03758814.2**

(22) Date of filing: **23.10.2003**

(51) Int Cl.7: **A61K 7/48**, A61K 7/00, A61K 7/40

(86) International application number:
**PCT/JP2003/013545**

(87) International publication number:
**WO 2004/039347 (13.05.2004 Gazette 2004/20)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **01.11.2002 JP 2002319841**

(71) Applicant: **Kao Corporation**
**Tokyo 103-8210 (JP)**

(72) Inventor: **NISHIO, Masaya,**
**KAO CORP. RESEARCH LABORATORIES**
**WAKAYAMA-SHI, Wakayama 640-8580 (JP)**

(74) Representative: **HOFFMANN EITLE**
**Patent- und Rechtsanwälte**
**Arabellastrasse 4**
**81925 München (DE)**

# (54) LIQUID SKIN PROTECTIVE COMPOSITION

(57) Provided is a liquid skin protective composition capable of protecting the skin from chemical substances in the environment and aqueous irritants such as body fluids, wastes, and exudates, causing less irritation, being applicable easily to the skin, and giving a good skin feel.

The liquid skin protective composition contains the below-described ingredients (A) and (B) and having a water content not more than 10 wt.%:

(A) a block copolymer having a linear polysiloxane-polyoxyalkylene block as a structural unit and having an HLB not more than 6,
(B) at least one oil selected from mineral oils, vegetable oils, animal oils, synthetic oils and organopolysiloxanes other than the ingredient (A).



Printed by Jouve, 75001 PARIS (FR)

## Description

### Technical Field

The present invention relates to a liquid skin protective composition. More specifically, the invention pertains to a liquid skin protective composition to be used for protecting the skin from any substances having an adverse effect on the skin when the skin comes into contact with the substances such as chemical substances in the environment, chemical substances in water, allergens, urine, feces, sweat, blood, body fluid, and wound exudate, particularly for protecting the skin at a diapered site of babies or other incontinent individuals, the skin around stoma and the skin around wound with exudate.

### Background Art

The surface of the skin is covered with a horny cell layer. Ceramide, lipid, NMF and the like contained in the horny cell layer serve as a barrier against foreign matters from the outside world and they are highly effective for blocking permeation of these foreign matters. It is however known that since the skin, inside of a diaper, of infants or care receivers using diapers, the skin around stoma or the skin around wounds with exudate such as bedsores tend to be in contact for long hours with a high water-content liquid such as sweat, urine, feces, wound exudate or the like, the barrier function of the skin is destroyed and lowered by the action of water, enzymes such as lipase, protease and urease, microorganisms, ammonia generated thereby, or toxins. Feces excreted in a diaper stick to the skin. They must be removed by vigorously scrubbing the skin or performing genital washing upon each diaper change. Such a behavior increases a care-giver burden and in addition, repeated genital scrubbing or washing whenever a diaper is changed damages the skin which has already lowered in resistance and moreover, increased in friction resistance owing to the influence of maceration resulting from the friction of the skin with a towel or paper used for scrubbing or washing. These are said to be causes of diaper rash. They are also said to be causative of bedsores in the bedridden elderly.

A skin protectant using an alkylsiloxane-containing polymer (for example, JP-A-4-501076), a skin protectant using an organic silicone resin (for example, JP-A-61-65808) and a skin protectant making use of a silicone/acrylic copolymer (for example, JP-A-2-262512) show high adhesion to the skin, but their use for the delicate skin is not preferred because they must contain an organic solvent which causes severe skin irritation. In addition, they are released from the skin easily under the conditions inducing perspiration. Under dry conditions, on the contrary, they are highly adhesive to the skin, but use of a releasing agent which causes severe irritation is inevitable for their release. Moreover, they have a problem such as insufficient skin protecting effect, because a uniform film cannot be formed on the surface such as skin which is uneven and does not stay still.

A foaming skin protectant (for example, JP-A-2002-145755) requires emulsification of its effective ingredient so that it contains a large amount of an emulsifier. In such a system, when a high-water-content substance such as urine or exudate adheres to the skin, re-emulsification occurs. This facilitates peeling of the protectant from the skin and a film effective for a prolonged period cannot be formed. There is also a report on a skin cosmetic composition containing a non-hydrolytic block copolymer having linear polysiloxane-polyoxyalkylene blocks as a repeating unit (JP-A-4-234307). This skin cosmetic composition contains a large amount of water so that it stimulates the skin by giving a cold feeling thereto upon application. In addition to such an uncomfortable feeling, it has a high viscosity, which prevents uniform application thereof over a wide area.

There is also a report of a skin protectant containing a film forming agent, a hydrophobic powder and a volatile oil (JP-A-7-242528). According to this literature, a large amount of ethanol is used as a solvent for the film forming ingredient. When such a skin protectant is applied to a delicate part of the skin such as rough skin, a smart pain is induced by ethanol. It is therefore utterly unsuited for use.

Skin protectants capable of satisfying all of the following conditions, that is, high skin-protecting effect, long lasting effect, easy application with less irritation, and a comfortable feeling after application have not yet been reported.

An object of the present invention is to provide a liquid skin protective composition which is capable of protecting the skin from chemical substances in the environment, or from aqueous irritants such as body fluids, waste and exudates, is readily applicable with less irritation and gives a good skin feel.

### Summary of the Invention

As a result of various investigations, the present inventor has found that a skin-protectant not only having an excellent skin-protecting effect but also giving a good skin feel, causing less irritation and being applied readily can be obtained by using a block copolymer having a linear polysiloxane-polyoxyalkylene block as a structural unit and having a specific HLB in combination with an oil, and by reducing the water content.

In the present invention, there is thus provided a liquid skin protective composition containing the below-described ingredients (A) and (B) and having a water content not more than 10 wt.%:

(A) a block copolymer having a linear polysiloxane-polyoxyalkylene block as a structural unit and having an HLB not more than 6;
(B) at least one kind of oil selected from mineral oils, vegetable oils, animal oils, synthetic oils and organopolysiloxanes other than the ingredient (A).

Best Mode for Carrying Out the Invention

Examples of the structure of the polyoxyalkylene block portion of the ingredient (A) of the present invention, that is, the block copolymer having a linear polysiloxane-polyoxyalkylene block as a structural unit include polyoxyethylene, polyoxypropylene, and polyoxyethylene polyoxypropylene. Of these, polyoxyethylene polyoxypropylene is more preferred, because it permits uniform dissolution when a water repellent oil or volatile oil is added, and it has excellent low-temperature stability.
As the ingredient (A), the linear polysiloxane-polyoxyalkylene block polymer as described in JP-A-4-234307 can be used. As the block copolymer serving as the ingredient (A), usable is a linear polysiloxane-polyoxyalkylene block copolymer represented by the following formula (1):

$$\left( -\!\!\left[ Y(R_2SiO)_aR_2SiYO \right]\left[ AO \right]_b - \right)_c \qquad (1)$$

(wherein, R represents a monovalent hydrocarbon group free of an aliphatic unsaturated group, A represents an alkylene group having from 2 to 4 carbon atoms, b stands for a number of from 4 to 100 (preferably from 10 to 90), b pieces of AO may be the same or different, c stands for a number of from 2 to 40 (preferably from 2 to 20), a stands for a number of from 4 to 100 (preferably from 5 to 40), and Y represents a divalent organic group bound to the adjacent silicon atom via a carbon-silicon bond and to a polyoxyalkylene block via an oxygen atom).
In particular, good results can be achieved by the use of a high-molecular-weight non-hydrolytic block copolymer having, as a structural unit, a linear polysiloxane-polyoxyalkylene block represented by the following formula (2):

$$-\!\!\left[ (R''_2SiO)_{a+1}SiR''_2CH_2CR'HCH_2-O(AO)_bCH_2CR'HCH_2 \right]_c\!\!- \qquad (2)$$

(wherein, a stands for a number of from 4 to 100 (preferably, a number of from 5 to 40), b stands for a number of from 4 to 100 (preferably, a number of from 10 to 90), b pieces of AO may be the same or different, c stands for a number of from 2 to 40 (preferably, a number of from 2 to 20), R' represents a monovalent hydrocarbon group and R" represents a monovalent hydrocarbon group) available by reacting a compound represented by the following formula (A) :

$$HR''_2SiO(R''_2SiO)_aSiR''_2H \text{ (A)} \qquad \text{(A)}$$

(wherein, R" represents a monovalent hydrocarbon group free of an aliphatic unsaturated group, a stands for a number of from 4 to 100 (preferably, a number of from 5 to 40) with a compound represented by the following formula (B):

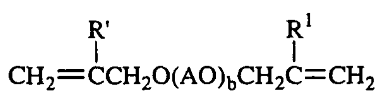
(B)

(wherein, R' represents a monovalent hydrocarbon group, A represents an alkylene group having from 2 to 4 carbon atoms, b stands for a number of from 4 to 100 (preferably from 10 to 90), and b pieces of AO may be the same or different).
Examples of R, R' and R" in the above-described formulas include groups selected from alkyl groups (such

as methyl, ethyl, propyl, isopropyl, butyl, pentyl, hexyl, octyl, decyl, dodecyl, octadecyl and eicosyl), aryl groups (such as phenyl and naphthyl), aralkyl groups (such as benzyl and phenylethyl), tolyl group, xylyl group, and cyclohexyl group. In the above-described formulas, examples of the divalent organic group represented by Y include -R'''-, -R'''-CO-, -R'''-NHCO-, -R'''-NHCONH-R''''-NHCO-, and -R'''-OOCNH-R''''-NHCO- (in which, R''' represents a divalent alkylene group such as ethylene, propylene or butylene, R'''' represents a divalent alkylene group such as R''' or a divalent arylene group such as - $C_6H_4$-, -$C_6H_4$-$C_6H_4$-, -$C_6H_4$-$CH_2$-$C_6H_4$-, or $C_6H_4$-$CH(CH_3)_2$-$C_6H_4$-, with a phenylene group being preferred as R''''). More preferred examples of the divalent organic group include - CH2CH2-, -CH2CH2CH2-, -$CH_2CH_2CH_2CH_2$-, -$(CH_2)_2$CO-, -$(CH_2)_3$NHCO-, -$(CH_2)_3$NHCONH$C_6H_4$NHCO- and -$(CH_2)_3$OOCN$C_6H_4$NHCO-. Of these, divalent alkylene groups are most preferred as Y, with - $CH_2CH_2CH_2$- being especially preferred.

The above-described non-hydrolytic copolymer can be prepared by reacting a polyoxyalkylene compound having a reactive terminal group with a dihydrocarbylsiloxane liquid having a terminal group reactive with the reactive terminal group of this polyoxyalkylene compound.

The above-described polyoxyalkylene compounds include polyoxyethylene, polyoxypropylene, polyoxybutylene, and polyoxyethylene-oxypropylene mixture, of which the polyoxyethylene-oxypropylene mixture is more preferred because it exhibits excellent stability when mixed.

The most preferred examples include block copolymers represented by the following formula:

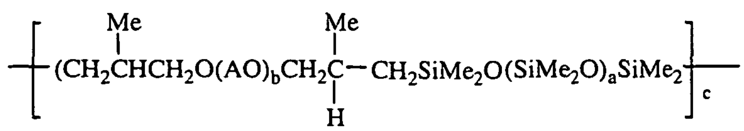

obtained by reacting a polyoxyalkylene compound having at each end thereof a $CH_2$=$C(CH_3)$-$CH_2$- group with a dimethylpolysiloxane having at each end thereof an $HSi(CH_3)_2O$- group. In the above-described formula, Me represents a methyl group, A represents an alkylene group having from 2 to 4 carbon atoms, b stands for a number of from 4 to 100 (preferably from 10 to 90), b pieces of AO may be the same or different, a stands for a number of from 4 to 100 (preferably from 5 to 40) and c stands for a number of from 2 to 40 (preferably from 2 to 20).

The block copolymer having the linear polysiloxane-polyoxyalkylene block as a recurring unit must have an HLB not more than 6. When the block copolymer has an HLB not more than 6, it does not dissolve away by emulsification upon contact with water and has high water resistance, and at the same time, is effective for preventing permeation of an irritant through the skin, thus having a skin-protecting effect. The HLB is preferably not more than 4 from the standpoint of further improving the water resistance further. The HLB can be determined in the following manner.

After measurement of a cloud number A, the HLB is determined in accordance with the below-described conversion formula. The term "cloud number A" as used herein means the number of mL of a 2% aqueous phenol solution required for titration of a solution (when the liquid becomes turbid, the titration is terminated) which has been obtained by dissolving 0.5 g of a block copolymer having a linear polysiloxane-polyoxyalkylene block as a recurring unit in 5 ml of ethanol, while maintaining the solution at 25°C.

$$\text{HLB} = 0.89 \times (\text{cloud number A}) + 1.11$$

When the water content in the liquid skin protective composition is adjusted to not more than 10 wt.%, the ingredient (A) brings about such an effect that the liquid skin protective composition is in the liquid form and spreads well when it is applied to the skin. The composition then thickens or turns into a gel, upon absorbing water evaporated from the skin or from the environment, whereby diffusion of the composition over the skin is suppressed and its effect lasts long. In addition, the polyoxyalkylene block and the skin surface interact with each other, for example, by forming a hydrogen bond so that the composition has a higher adhesive power to the skin than an ordinary baby oil composed of liquid paraffin. It is not peeled off easily by friction so that it can protect the skin from friction with cloth or the like. Moreover, since the ingredient (A) is not solid, it does not bring discomfort caused by impeding the movement of the skin. The ingredient (A) is less sticky and very easily applicable to the skin. Even if the skin is wet, the ingredient (A) can be applied without any difficulty and exhibits its skin protective effect.

The content of the ingredient (A) in the liquid skin protective composition is preferably from 3 to 50 wt.%, especially preferably from 5 to 20 wt.%.

As the ingredient (B) of the present invention, at least one kind of oil selected from mineral oils, animal oils, vegetable oils, synthetic oils and organopolysiloxanes other than the ingredient (A) can be used. Owing to their emollient effects for the skin, these oils have moisturizing effects and are effective for preventing excessive drying of the skin.

In addition, they can supplement skin lipid ingredients and serve to reinforce the skin barrier function. They improve the water repellency and water resistance of the skin protectant. Moreover, owing to the lubricating effect, they can reduce the friction on the skin surface.

Specific examples include mineral oils such as mineral oil, liquid paraffin, liquid isoparaffin, naphthenic oil and petrolatum, vegetable oils such as olive oil, jojoba oil, cotton seed oil, peanut oil, castor oil, sunflower oil, coconut oil, almond oil, palm oil, safflower oil, corn oil, soybean oil, hydrogenated castor oil and hydrogenated palm oil; animal oils such as mink oil, turtle oil, guinea pig oil, squalane, beef tallow and lanolin; and synthetic oils such as poly-$\alpha$-olefin oil, polyalkylene glycol oil, polybutene oil, and ester oils such as isopropyl myristate, dioctyl phthalate, diethylhexyl phthalate, octyldodecyl myristate, myristyl myristate, glycerin-tri-2-ethylhexyl and triglyceride stearate. Examples of the organopolysiloxane include dimethylpolysiloxane, methylphenylpolysiloxane, silicone gum, alkyl-modified methylpolysiloxane, alkoxy-modified methylpolysiloxane and amino-modified methylpolysiloxane. These oils may be used either singly or in combination of two or more of them.

The content of the ingredient (B) in the liquid skin protective composition is preferably not less than 10 wt.%, more preferably from 30 to 97 wt.%, especially preferably from 50 to 95 wt.%.

The ingredient (B) preferably contains (B-1) an oil ingredient having a volatility of not less than 0.2 $mg/cm^2hr$ and (B-2) an oil ingredient having a volatility not more than 0.1 $mg/cm^2hr$. It is more preferred that the ingredient (B-2) contains two kinds of oil having a volatility not more than 0.1 $mg/cm^2hr$ and mutual solubility not more than 10 wt.%.

The ingredient (B-1) has a low viscosity and easily spreads on the skin upon application so that it has an effect of reducing stickiness and improving the skin feel. The ingredient (B-2) is useful for maintaining the water resistance of the liquid skin protective composition of the invention. The ingredient (B-1) preferably has a higher volatility, because the diffusion of the skin protective composition over the skin can be suppressed by the volatilization of the ingredient (B-1) and the composition is effective for longer hours and in addition, provides an improved skin feel. The volatility can be measured as a loss in weight per unit area and unit hour when 10 g of an oil is put into a Petri dish having an inner diameter of 85 mm and depth of 11 mm and allowed to stand at 25°C under atmospheric pressure. The volatility of the ingredient (B-1) is preferably from 0.2 to 50 $mg/cm^2hr$, more preferably from 0.3 to 20 $mg/cm^2hr$, especially preferably from 0.5 to 5 $mg/cm^2hr$.

The ingredient (B-2) is preferably added in order to keep water resistance and lubrication of the skin protectant of the invention even after evaporation of the ingredient (B-2) when the protectant is applied to the skin. The lower the volatility of the ingredient (B-2) is the longer the skin-protecting effect is maintained. The volatility at 25°C is preferably not more than 0.1 $mg/cm^2hr$, more preferably not more than 0.03 $mg/cm^2hr$.

Examples of the ingredient (B-1) include volatile organopolysiloxanes and volatile liquid isoparaffin. The volatile organopolysiloxanes include hexamethyldisiloxane, octamethyltrisiloxane, decamethyltetrasiloxane, octamethylcyclotetrasiloxane and decamethylcyclopentasiloxane. Use of cyclic methylcyclopolysiloxanes such as decamethylcyclopentasiloxane is preferred in consideration of less irritation and good skin feel when a composition containing it is sprayed. The content of the ingredient (B-1) in the liquid skin protective composition is not less than 10 wt.%, more preferably from 30 to 80 wt.%.

Preferred ingredient (B-2) includes at least one selected from liquid paraffin, liquid isoparaffin, squalane, isopropyl myristate, fatty acid triglyceride, decamethylcyclopentasiloxane, methylpolysiloxane, methylcyclopolysiloxane and dimethylpolysiloxane. Of these, at least one selected from liquid paraffin, liquid isoparaffin, squalane, methylcyclopolysiloxane and dimethylpolysiloxane is more preferred.

The ingredient (B-2) preferably contains two kinds of oil having a mutual solubility not more than 10 wt.%. At a low mutual solubility, when the composition is applied to the skin, non-sticky feel remains long after evaporation of the ingredient (B-1), which brings about a high lubrication effect. The term "mutual solubility" means a lower concentration of the concentrations of one of the two kinds of oil (wt.% in the mixture) at the time when the mixture obtained by adding under stirring one of the two kinds of oil to the other turns from transparent to turbid. The mutual solubility at 25°C is preferably not more than 10 wt.%, more preferably not more than 3 wt.%, especially preferably not more than 1 wt.%. It is preferred to use such two kinds of oil at a mixing ratio (wt/wt) of from 1/4 to 4/1, more preferably from 1/2 to 2/1. Examples of such a combination include a combination of a hydrocarbon oil having a relatively large molecular weight such as liquid paraffin or squalane and an organopolysiloxane such as dimethylpolysiloxane. These ingredients can be added stably by the use of the above-described volatile organopolysiloxane or volatile liquid isoparaffin. When the volatile ingredient evaporates, phase separation of these ingredients occur, which leads to exhibition of effects.

In order to attain high lubrication, the viscosity at 25°C of the hydrocarbon oil is preferably not less than 20 mPa·s and the viscosity at 25°C of the organopolysiloxane is preferably not less than 10 mPa·s. The viscosity at 25°C of the hydrocarbon oil is more preferably not less than 40 mPa·s and the viscosity at 25°C of the organopolysiloxane is more preferably not less than 20 mPa·s.

The content of the ingredient (B-2) in the liquid skin protective composition is preferably not less than 10 wt.%, more preferably from 20 to 80 wt.%, especially preferably from 30 to 60 wt.%.

The water content in the liquid skin protective composition of the invention is not more than 10 wt.%, preferably not more than 3 wt.%, more preferably not more than 1 wt.%. When the water content in the composition is not more than 10 wt.%, the viscosity of the whole composition lowers, facilitating application of the composition. In this case, addition of a surfactant or emulsifier is not necessary and the composition does not melt away over the skin by emulsification so that it has improved water resistance and becomes less irritant to the skin. Application of water to an area of the skin sensitive to stimulation is not preferred, because water gives a cold feel to the skin owing to a considerably large latent heat of evaporation. When the water content is not more than 3 wt.%, a cold feel given to the skin is very weak. At a water content not more than 1 wt.%, the cold feel approaches zero. In addition, the composition having a smaller water content is effective for reducing the friction on the skin.

The liquid skin protective composition of the invention is preferably in the liquid form and has a low viscosity at normal temperature (25°C). The viscosity at 25°C is preferably not more than 70 mPa·s, more preferably not more than 50 mPa·s, especially not more than 30 mPa·s. When the composition has a low viscosity, it can be spread over the skin readily upon application. It can also be applied over a wide area by spraying.

In the liquid skin protective composition of the present invention, the ingredient (A) and ingredient (B) are included at a weight ratio (A)/(B) of from 3/100 to 60/100, more preferably from 3/100 to 30/100, especially preferably from 5/100 to 15/100. When the ratio is not less than 3/100, the resulting composition has an improved skin protecting effect. When the ratio is not more than 60/100, on the other hand, the resulting composition has a reduced viscosity, has improved applicability and provides a good skin feel after application.

The content of the monovalent alcohol having from 1 to 4 carbon atoms in the liquid skin protective composition of the present invention is preferably not more than 10 wt.%. Examples of the monovalent alcohol having from 1 to 4 carbon atoms include ethanol, methanol, isopropanol, 1-propanol and butyl alcohol. The content of the monovalent alcohol having from 1 to 4 carbon atoms is more preferably not more than 3 wt.%, especially preferably not more than 1 wt.%. When the content of the volatile alcohol in the composition of the present invention is not more than 10 wt.%, the composition is mild to the skin, for example, the composition does not smart, and moreover, continuous use of the composition does not roughen the skin even if it is applied to a very delicate area of the skin, for example, buttocks or the skin around a stoma or a wound. Application of the composition of the present invention to these delicate areas is mainy intended in the present invention. In particular, when the content is not more than 3 wt.%, an irritating feeling given to the skin upon application lowers further and in addition, a cold feeling which is caused by the latent heat of evaporation of a volatile ingredient becomes less. The composition is almost free from giving an irritating feeling or a cold feeling upon application particularly when the content is not more than 1 wt.%.

The liquid skin protective composition of the present invention may further contain an anti-inflammatory agent. Incorporation of the anti-inflammatory agent enables to prevent occurrence of inflammation such as diaper rash, redness, contact-type dermatitis or the like. Even if the content of the anti-inflammatory agent is small, it remains long on the skin and is expected to provide a high effect when incorporated in the liquid skin protective composition. Although no particular limitation is imposed on the anti-inflammatory agent, examples include guaiazulene, allantoin, soybean lecithin, anethole, linalyl acetate, terpinyl acetate, glycyrrhetic acid, stearyl glycyrrhetinate, dipotassium glycyrrhetinate, and hydrocortisone acetate. Anti-inflammatory agents having a high lipophilic property such as guaiazulene are preferred from the viewpoint of mixing with the other ingredients. Its content in the liquid skin protective composition is not more than 1 wt.%, preferably from 0.0001 to 0.2 wt.%, more preferably from 0.0005 to 0.1 wt.%.

Since the liquid skin protective composition of the present invention is applied to an area which may probably contact with microbe causing skin inflammation (such microbe may be called "infecting organisms") in feces or wound exudates, occurrence of infectious diseases such as urinary tract infection due to such infecting organisms can be prevented by incorporating an antimicrobial agent in the composition. Although no particular limitation is imposed on the antimicrobial agent, those causing as less skin irritation as possible are preferred because antimicrobial agents are known to cause skin irritation. In addition, those having a high lipophilic property are preferred from the standpoint of stability upon mixing. Specific examples include isopropylmethylphenol, triclosan, benzalkonium chloride, benzethonium chloride and chlorhexidine gluconate. Its content, as an effective ingredient, in the composition of the present invention is from 0.0001 to 1 wt.%, preferably from 0.0005 to 0.2 wt.%, more preferably from 0.001 to 0.05 wt.%.

The liquid skin protective composition of the present invention is preferably free of an inorganic powder such as pigment. When the composition does not contain an inorganic powder, the resulting liquid skin protective composition can be released from the skin by washing, has high lubrication effect and does not clog a nozzle when used in the form of a spray.

To the liquid skin protective composition of the present invention may be added, as necessary, a humectant, an antioxidant, a blood circulation promoter, an antiseptic, a deodorant, an ultraviolet absorber, a colorant, a pigment and the like to an extent, in term of both quantity and quality, not impairing the advantages of the present invention.

The liquid skin protective composition of the present invention can be prepared in a manner known per se in the art by adding to the above-described essential ingredients the above-described optional ingredients, if necessary. These optional ingredients can be added to the composition in an amount not more than 30 wt.%.

The liquid skin protective composition of the present invention can be provided in any form of liquid, mist and foam. It can be used, for example, in the form of an aerosol spray, pump spray, pump bottle or tube, or absorbent cotton, paper or cloth impregnated with it. A mist spray or nonwoven cloth impregnated with the composition is desired because it can be applied to a wide area without direct contact. Application of the composition by spray does not need direct contact with the affected area so that hand washing after use can be omitted. Use of a pump spray container without a propellant such as liquefied gas or carbon dioxide gas is most preferred, because stimulation or a cold feeling to the skin which will otherwise be brought by the evaporation heat of the propellant can be reduced. The composition can be applied readily to a wide area by using a nonwoven cloth impregnated with the composition and when the composition is applied in such a manner, there will be no possibility of staining floor caused by dripping or scattering of the composition.

Examples

Examples 1 to 7, Comparative Examples 1 to 4

Liquid skin protective compositions of Examples 1 to 7 and Comparative Examples 1 to 4 having compositions (on a weight basis) as shown in Tables 1 and 2 were prepared in a manner known per se in the art in accordance with the below-described formulations.

Evaluation method

[Viscosity]

Viscosity was measured using a Brookfield type viscometer at 25°C.

[Evaluation of skin protecting effect]

The color of the skin was measured using a colorimeter ("ND300A", product of Nippon Denshoku Industries Co., Ltd., spectrophotometer) and the liquid skin protective composition was applied to the site of the skin. A 0.5% aqueous solution of Blue No. 1 was then brought into contact for 5 minutes with the skin to which the liquid skin protective composition had been applied. After the liquid skin protective composition was washed away, the color of the skin was measured by the colorimeter. A color difference ($\Delta E$) between the skin after the treatment, and the skin before application of the liquid skin protective composition and before contact with the colorant solution was determined in accordance with JIS Z8730. How much coloring of the skin was prevented compared with the color difference ($\Delta E_0$) when the colorant solution was brought into contact with a site to which nothing had been applied can be calculated as a skin protecting effect (%) from the below-described equation.

$$\text{Skin protecting effect (\%)} = (\Delta E_0 - \Delta E)/\Delta E_0 \times 100$$

As the percentage approaches 100%, a skin-protecting effect becomes higher and better.

[Evaluation of friction coefficient]

The liquid skin protective composition was applied with a coating weight of 3 mg/cm$^2$ to a polyurethane foam ("OK mat", product of Okamoto Co., Ltd.) used as a model skin. After it was allowed to stand at room temperature for not less than 2 hours, friction between the model skin and a cotton fabric (Kanakin No. 3), that is, a standard sample made by Japanese Standards Association was measured using a KES surface friction property tester (product of Kato Tech Co., Ltd.), whereby a friction coefficient was obtained.

(Evaluation using a pump-type spray container under natural pressure)

The liquid skin protective composition of each of Examples 1 to 6 and Comparative Examples 1 to 4 was filled in a pump type spray container capable of spraying the composition in the form of a mist and a panel of 13 caregivers applied the composition to the buttock skin of bedridden care receivers after their consent. The applicability and skin protecting effect were evaluated by the organoleptic evaluation of the caregivers, while the irritating feeling and skin feel were evaluated based on the results of interview with the care receivers. Thus, the performances of the composition were evaluated.

The liquid skin protective composition was sprayed through a pump type spray container in an amount of about 0.2 g per one spray and it was sprayed about four times to the buttock skin.

(Evaluation using a sheet obtained by impregnating a nonwoven fabric with the skin protective composition)

A cotton nonwoven spunlace ("Cotton Ace", product of UNITIKA Ltd., basic weight: 40 g) was impregnated with the liquid skin protective composition of Example 7. The amount of the liquid skin protective composition was 150% of the weight of the nonwoven fabric. Each sheet had a size of 150 mm × 190 mm. One box was filled with 40 sheets.

Evaluation results are shown in Tables 1 and 2. The number in the column of the evaluation results means the number of persons.

Table 1

| | | | Examples | | | | | | |
|---|---|---|---|---|---|---|---|---|---|
| | | | 1 | 2 | 3 | 4 | 5 | 6 | 7 |
| Ingredient (A) | Block copolymer having a linear | HLB=1 [1)] | 10 | | 5 | | | 10 | 10 |
| | polysiloxane-polyoxyalkylene block | HLB=3 [2)] | | 10 | | 35 | 20 | | |
| | as a recurring unit | HLB=7 [3)] | | | | | | | |
| Ingredient (B) | Methyl polysiloxane 1 [4)] | | | 45 | | | | | |
| | Methylpolysiloxane 2 [5)] | | | | | | | 20 | 20 |
| | Liquid paraffin 1 [6)] | | | | 20 | | | | |
| | Liquid paraffin 2 [7)] | | | | | | | 20 | 20 |
| | Squalane | | | | 25 | | | | |
| | Safflower oil | | | | | | 46 | | |
| | Isopropyl myristate [8)] | | 54 | | | | | | |
| | Fatty acid triglyceride [9)] | | | | | 25 | | | |
| | Decamethylcyclopentasiloxane [10)] | | 36 | | 50 | | 30 | 50 | 50 |
| | Liquid isoparaffin [11)] | | | 40 | | | | | |
| | Organic silicone resin / octamethylcyclopolysiloxane (weight ratio 50/50) [12)] | | | | | | | | |
| | Methylpolysiloxane 1.5cs [13)] | | | | | 40 | | | |
| | Methylphenylsilicone [14)] | | | | | | | | |
| Water | | | | 4.99 | | | | | |
| Ethanol | | | | | | | 3.979 | | |
| Dipotassium glycyrrhizinate [15)] | | | | 0.01 | | | | | |
| α-Tocopherol acetate | | | | | | | 0.2 | | |
| Benzalkonium chloride [16)] | | | | | | | 0.001 | | |
| Viscosity (mPa ·s) | | | 12 | 26 | 11 | 62 | 30 | 20 | 20 |
| Skin protecting effect (%) | | | 88 | 91 | 82 | 89 | 83 | 89 | 89 |
| Friction coefficient | | | 4.6 | 5.4 | 5.8 | 4.8 | 6.1 | 3.1 | 3.1 |
| Applicability | | Good | 11 | 10 | 12 | 8 | 12 | 12 | 10 |
| | | Poor | 2 | 3 | 1 | 5 | 1 | 1 | 3 |

Table 1 (continued)

| | | Examples | | | | | | |
|---|---|---|---|---|---|---|---|---|
| | | 1 | 2 | 3 | 4 | 5 | 6 | 7 |
| Irritation upon application | Absent | 13 | 8 | 13 | 12 | 7 | 13 | 12 |
| | Present | 0 | 5 | 0 | 1 | 6 | 0 | 1 |
| Skin feel after application | Good | 12 | 9 | 11 | 7 | 10 | 12 | 12 |
| | Poor | 1 | 4 | 2 | 6 | 3 | 1 | 1 |
| Skin protecting effect | Present | 9 | 9 | 6 | 11 | 10 | 10 | 9 |
| | Slightly present | 4 | 3 | 6 | 2 | 2 | 3 | 4 |
| | None | 0 | 1 | 1 | 0 | 1 | 0 | 0 |

Table 2

| | | | Comparative Examples | | | |
|---|---|---|---|---|---|---|
| | | | 1 | 2 | 3 | 4 |
| Ingredient (A) | Block copolymer having a linear | HLB=1 1) | | | | |
| | polysiloxane-polyoxyalkylene block as a | HLB=3 2) | | | 10 | |
| | recurring unit | HLB=7 3) | 10 | | | |
| Ingredient (B) | Methyl polysiloxane 1 4) | | | | 35 | |
| | Methylpolysiloxane 2 5) | | | | | |
| | Liquid paraffin 1 6) | | | | | |
| | Liquid paraffin 2 7) | | | | | |
| | Squalane | | | | | |
| | Safflower oil | | | | | |
| | Isopropyl myristate 8) | | 54 | 60 | | |
| | Fatty acid triglyceride 9) | | | | | |
| | Decamethylcyclopentasiloxane 10) | | 36 | 40 | | |
| | Liquid isoparaffin 11) | | | | 40 | |
| | Organic silicone resin / octamethylcyclopolysiloxane (weight ratio 50/50) 12) | | | | | 10 |
| | Methylpolysiloxane 1.5cs 13) | | | | | 85 |
| | Methylphenylsilicone 14) | | | | | 5 |
| Water | | | | | 14.99 | |
| Ethanol | | | | | | |
| Dipotassium glycyrrhizinate 15) | | | | | 0.01 | |
| α-Tocopherol acetate | | | | | | |
| Benzalkonium chloride 16) | | | | | | |
| Viscosity (mPa·s) | | | 12 | 6 | 138 | 8 |
| Skin protecting effect (%) | | | 10 | 17 | 70 | 23 |

Table 2 (continued)

| | | Comparative Examples | | | |
|---|---|---|---|---|---|
| | | 1 | 2 | 3 | 4 |
| Friction coefficient | | 7.3 | 6.3 | 8.3 | 6.5 |
| Applicability | Good | 11 | 12 | 0 | 10 |
| | Poor | 2 | 1 | 13 | 3 |
| Irritation upon application | Absent | 11 | 12 | 4 | 8 |
| | Present | 2 | 1 | 9 | 5 |
| Skin feel after application | Good | 3 | 11 | 2 | 3 |
| | Poor | 10 | 2 | 11 | 10 |
| Skin protecting effect | Present | 1 2 4 | | | 4 |
| | Slightly present | 4 | 6 | 6 | 5 |
| | None | 8 | 5 | 3 | 4 |

In Tables 1 and 2:

1): "SIL-WET FZ-2203", trade name; product of Nippon Unicar Co., Ltd. Structural unit:

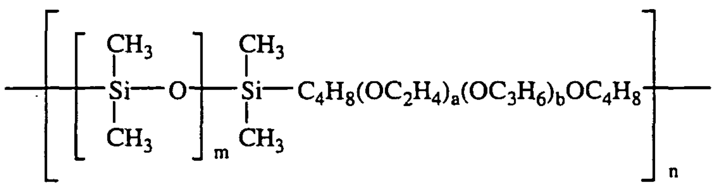

2) "SIL-WET FZ-2207", trade name; product of Nippon Unicar Co., Ltd. Structural unit:

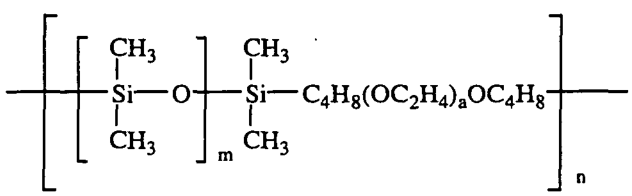

3) "SIL-WET FZ-2222", trade name; product of Nippon Unicar Co., Ltd. Structural unit:

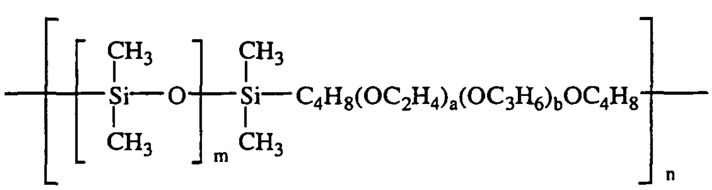

4) "SH200C 10cs", trade name; product of Dow Corning Toray Silicone Co., Ltd.
5) "SH200C 20cs", trade name; product of Dow Corning Toray Silicone Co., Ltd.
6) "Hicall K-230", trade name; product of Kaneda Corporation.

7) "Hicall K-350", trade name; product of Kaneda Corporation.
8) "EXCEPARL IPM", trade name; product of Kao Corporation
9) "COCONARD MT", trade name; product of Kao Corporation
10) "SH245", trade name; product of Dow Corning Toray Silicone Co., Ltd.
11) "IP Solvent 1620", trade name; product of Idemitsu Petrochemical Co., Ltd.
12) "KF7312F", trade name; product of Shin-etsu Chemical Co., Ltd.
13) "SH200C 1.5cs", trade name; product of Dow Corning Toray Silicone
14) "KF56", trade name; product of Shin-etsu Chemical Co., Ltd.
15) "CO glychinol", trade name; product of Maruzen Pharmaceuticals Co., Ltd.
16) "Nissan Cation M2-100", trade name; product of NOF Corporation

The results shown in Tables 1 and 2 indicate that the compositions of Examples 1 to 6 are sprayed well at room temperature, the compositions of 1 to 7 do not give an irritating feeling upon application, give a very good skin feel with smoothness and can protect the skin very effectively. On the other hand, the composition of Comparative Example 1 exhibits a considerably reduced skin-protecting effect. The composition of Comparative Example 2 exhibits good applicability and skin feel, but exhibits only a low skin-protecting effect. The composition of Comparative Example 3 cannot be sprayed well and in addition, cannot be applied to the skin easily and gives an uncomfortable skin feel. Moreover, its skin-protecting effect is very low. The composition of Comparative Example 4 leaves a taut feeling and stickiness on the skin after application, does not give a good skin feel, and does not bring about an apparent skin-protecting effect.

Industrial Applicability

The liquid skin protective composition of the present invention has a high skin-protecting effect, can be applied to the skin easily, does not cause a skin irritation, and gives a good skin feel. It can therefore be used for protecting the skin, particularly, a diapered site of babies or incontinent patients, the skin around stoma, and the skin around wound with exudates.

## Claims

1. A liquid skin protective composition comprising the below-described ingredients (A) and (B) and having a water content not more than 10 wt.%:

   (A) a block copolymer having a linear polysiloxane-polyoxyalkylene block as a structural unit and having an HLB not more than 6,
   (B) at least one kind of oil selected from mineral oils, vegetable oils, animal oils, synthetic oils and organopolysiloxanes other than the ingredient (A).

2. The liquid skin protective composition of Claim 1, wherein the ingredient A is a block copolymer represented by the following formula (1):

$$\left( -\!\!\left[ Y(R_2SiO)_aR_2SiYO \right]\!\left[ AO \right]_b - \right)_c \qquad (1)$$

   (wherein, R represents a monovalent hydrocarbon group free of an aliphatic unsaturated group, A represents an alkylene group having from 2 to 4 carbon atoms, b stands for a number of from 4 to 100, b pieces of AO may be the same or different, c stands for a number of from 2 to 40, a stands for a number of from 4 to 100, and Y represents a divalent organic group bound to the adjacent silicon atom via a carbon-silicon bonded and to the polyoxyalkylene block via an oxygen atom).

3. The liquid skin protective composition of Claim 1 or 2, wherein the siloxane blocks each has an average molecular weight of from 500 to 10,000, the polyoxyalkylene blocks each has an average molecular weight of from 300 to 10,000, the siloxane blocks constitute from 10 to 90 wt.% of the copolymer, and the block copolymer has an average molecular weight of at least 3,000.

4. The liquid skin protective composition of any one of Claims 1 to 3, wherein the viscosity at 25°C is not more than

70 mPa·s.

**5.** The liquid skin protective composition of any one of Claims 1 to 4, wherein the ingredient (A) and the ingredient (B) are included at a (A)/(B) weight ratio of from 3/10 to 60/100.

**6.** The liquid skin protective composition of any one of Claims 1 to 5, wherein the content of the ingredient (A) is from 3 to 50 wt.%.

**7.** The liquid skin protective composition of any one of Claims 1 to 6, wherein the content of the ingredient (B) is not less than 10 wt.%.

**8.** The liquid skin protective composition of any one of Claims 1 to 7, wherein the ingredient (B) is at least one selected from liquid paraffin, liquid isoparaffin, methylcyclopolysiloxane and dimethylpolysiloxane.

**9.** The liquid skin protective composition of any one of Claims 1 to 8, wherein the ingredient (B) comprises (B-1) an oil ingredient having a volatility of not less than 0.2 $mg/cm^2hr$ and (B-2) an oil ingredient having a volatility not more than 0.1 $mg/cm^2hr$.

**10.** The liquid skin protective composition of Claim 9, wherein the ingredient (B-1) comprising two kinds of oil having a volatility not more than 0.1 $mg/cm^2hr$ and a mutual solubility not more than 10 wt.%.

**11.** A method of applying a liquid skin protective composition of Claim 1 by spraying.

**12.** A method of applying a liquid skin protective composition of Claim 1 by using a material impregnated with the composition.

**13.** Use of a liquid skin protective composition of Claim 1 to protect the skin.

**INTERNATIONAL SEARCH REPORT**

International application No.
PCT/JP03/13545

A. CLASSIFICATION OF SUBJECT MATTER
Int.Cl[7] A61K7/48, 7/00, 7/40

According to International Patent Classification (IPC) or to both national classification and IPC

B. FIELDS SEARCHED

Minimum documentation searched (classification system followed by classification symbols)
Int.Cl[7] A61K7/00-7/50

Documentation searched other than minimum documentation to the extent that such documents are included in the fields searched

Electronic data base consulted during the international search (name of data base and, where practicable, search terms used)
WPI/L

C. DOCUMENTS CONSIDERED TO BE RELEVANT

| Category* | Citation of document, with indication, where appropriate, of the relevant passages | Relevant to claim No. |
|---|---|---|
| Y | EP 492657 A1 (NIPPON UNICAR CO., LTD.), 27 December, 1991 (27.12.91), Claims; page 2, lines 14 to 34; page 6, lines 2 to 7; examples & JP 4-234307 A Par. Nos. [0002] to [0004], [0033]; examples & US 5472686 A & EP 492657 B & JP 3071222 B & CA 2058461 A & DE 69116839 A1 & KR 158212 A | 1-13 |
| X | JP 7-277923 A (Kanebo, Ltd.), 24 October, 1995 (24.10.95), Claims; Par. No. [0001] (Family: none) | 1-13 |

[X] Further documents are listed in the continuation of Box C.   [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 January, 2004 (16.01.04) | 10 February, 2004 (10.02.04) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/13545

C (Continuation). DOCUMENTS CONSIDERED TO BE RELEVANT

| Category* | Citation of document, with indication, where appropriate, of the relevant passages | Relevant to claim No. |
|---|---|---|
| X | JP 9-95419 A (Nippon Unicar Co., Ltd.), 08 April, 1997 (08.04.97), Claims; Par. Nos. [0001], [0018]; examples 1 to 3, 5 to 12 (Family: none) | 1-13 |
| Y | JP 2000-63254 A (Kabushiki Kaisha Kanpehapio), 29 February, 2000 (29.02.00), Claims; Par. Nos. [0001], [0005], [0027] (Family: none) | 1-13 |
| Y | JP 7-233025 A (Kanebo, Ltd.), 05 September, 1995 (05.09.95), Claims; Par. Nos. [0001], [0003], [0034] (Family: none) | 1-13 |
| A | JP 2002-308729 A (Kansai Koso Kabushiki Kaisha), 23 October, 2002 (23.10.02), Par. Nos. [0002], [0021] (Family: none) | 1-13 |
| A | JP 9-315934 A (Kao Corp.), 09 December, 1997 (09.12.97), Claims; Par. No. [0031]; table 1; Par. No. [0048] (Family: none) | 1-13 |
| A | JP 6-72854 A (Kao Corp.), 15 March, 1994 (15.03.94), Claims; Par. Nos. [0001], [0024], [0038] (Family: none) | 1-13 |
| A | JP 6-72854 A (Kao Corp.), 15 March, 1994 (15.03.94), Claims; Par. Nos. [0001], [0026], [0032] (Family: none) | 1-13 |
| A | JP 5-262637 A (Kao Corp.), 12 October, 1993 (12.10.93), Claims; Par. Nos. [0001], [0003], [0026], [0037], [0038] (Family: none) | 1-13 |
| A | JP 7-238009 A (Kao Corp.), 12 September, 1995 (12.09.95), Claims; Par. Nos. [0001] to [0003], [0046], [0054] (Family: none) | 1-13 |
| A | EP 684041 A1 (L'OREAL), 29 November, 1995 (29.11.95), Claims & JP 7-316010 A & FR 2720274 A1 & DE 69500223 A1 | 1-13 |

Form PCT/ISA/210 (continuation of second sheet) (July 1998)